# EUROPEAN PATENT APPLICATION

(11) **EP 1 884 264 A1**
(43) Date of publication of application: **06.02.2008**
(21) Application number: 06016039.7
(22) Date of filing: 01.08.2006
(51) Int. Cl.: A63B 24/00

(54) **Method of programming an exercise apparatus**

(71) Applicant: Strength Master Fitness Tech Co., Ltd., Wufeng Township Taichung (TW)
(72) Inventor: Wu, Shen Yi, Wufeng Township Taichung (TW)
(74) Representative: Strehl Schübel-Hopf & Partner

(57) **Abstract**

A method of programming human electrical exercise apparatus comprises following steps: establishing an external information unit that contains a user's personal information, processing said information and outputting at least one exercise mode, and executing the exercise mode by selecting it to drive and operate the apparatus accordingly.
Part of the personal information are stored on a portable storage device.

## Description

### BACKGROUND OF THE INVENTION

### 1. Technical Field

The present invention relates to methods of programming human electrical exercise apparatus and more particularly, to a method of programming the apparatus with pre-established external information.

### 2. Description of Related Art

Contemporary apparatus are required to be equipped with versatile functions to satisfy the users' varied needs. As to an exercise treadmill, the running speed, inclination, and exercise time thereof may be subject to a user's programming for the desired exercise routine. Provided all the function variables have are programmed item-by-item, such complex and complicated settings significantly consumes the user's time and becomes a nuisance.

Besides, a user generally sets the function variables such as exercise time and running speed on a conventional exercise treadmill according to his past exercise experience but not physical condition. Thus, a need exists for a method of programming exercise apparatus in accordance with a user's individual physical fitness. Such a method must facilitate prompt programming exercise apparatus with an exercise mode that conforms to a user's individual exercise demand.

### SUMMARY OF THE INVENTION

The present invention has been accomplished under the circumstances in view. It is one object of the present invention to provide a method of programming human electrical exercise apparatus, which simply drives the apparatus with a pre-established external information unit instead of complex and complicated programming steps.

It is another object of the present invention to provide a method of programming electrical human exercise apparatus wherein the external information can be derived from a user's personal information and derives an individual exercise mode for the user.

To achieve these and other objects of the present invention, the method of programming human electrical exercise apparatus primarily comprises establishing an external information unit, processing the information and executing an exercise mode, wherein the details of these steps are;
establishing an external information unit - establishing an external information unit that contains a user's personal information;
processing the information - receiving said information for further processing and computing and outputting it as at least one exercise mode; and
executing an exercise mode - selecting an exercise mode to drive the exercise apparatus to operate accordingly.

### BRIEF DESCRIPTION OF THE DRAWINGS

FIG. 1 is a block diagram illustrating the programming method according to the present invention.
FIG. 2 is another block diagram illustrating the programming method according to the present invention.
FIG. 3 is a block diagram depicting the direct input and indirect input steps of the present invention.

### DETAILED DESCRIPTION OF THE PREFERRED EMBODIMENT

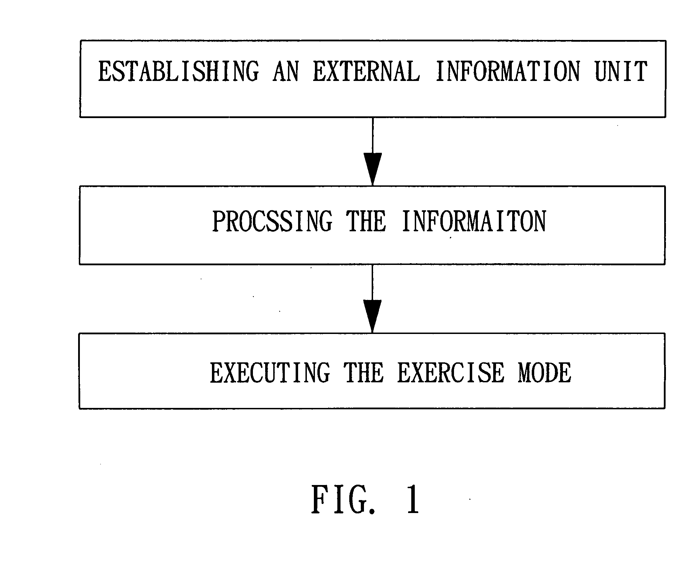

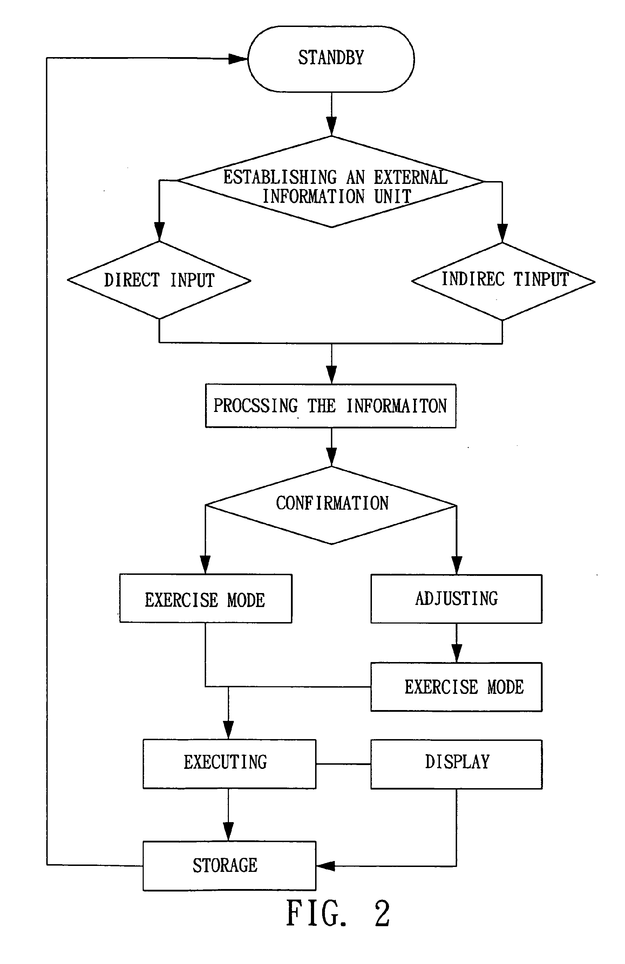

Referring to FIG. 1 and FIG. 2, according to one embodiment of the present invention, the method of programming human electrical exercise apparatus, for instance, an exercise treadmill comprises the steps of establishing an external information unit, processing the information and executing an exercise mode.

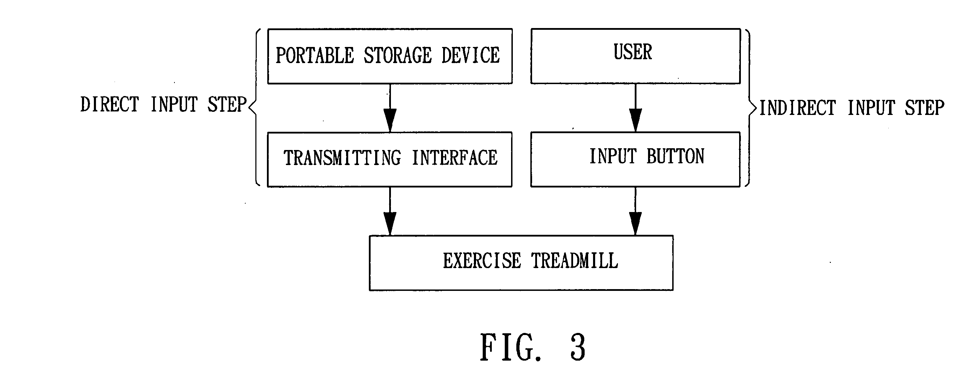

Said external information unit contains a user's personal information such as sex distinction, body weight, pulse rate, blood pressure value and age. To establish such external information unit, as shown in FIG. 3, the steps comprises an indirect input step and a direct input step wherein, in this embodiment, the indirect input step is related to inputting a user's personal information through an input button provided on a treadmill, and the direct input is related to inputting pre-established personal information of the user. Thereby, the user can optionally use either the direct input step or the indirect input step to provide his personal information to the apparatus, i.e. an exercise treadmill, for following processing.

In the same embodiment, the direct input of the external information is performed through a portable storage device, which is for the user's convenience in carrying, and a transmitting interface which can be an input socket installed on the treadmill. The portable storage device is loaded with pre-established user-information and the transmitting interface is applicable to read out the user's information therein for further processing.

Additionally, the portable storage device as stated above can be a SIM card, a USB flash disk or a memory card selected from the group consisting of SD card, MMC card, CF card, MS card, SM card and an XD card wherein a user's personal information is pre-loaded.

The next step is to process said information. While the personal information of the external information unit is received, it is simultaneously processed and transformed into at least one exercise mode for outputting. Hence, the user can program the treadmill by selecting the exercise mode or adjusting the exercise mode according to personal favor through an adjusting step.

The last step is to execute the selected exercise mode to operate the exercise apparatus correspondingly.

Thereupon, either the direct input or indirect input of the user's personal information can drive the treadmill to operate with the exercise mode most appropriate to the user's physical fitness. While the present invention provides a simple and fast programming method, it also gives consideration to the user's exact exercise demand to escape from excessive or insufficient exercise.

The aforesaid excessive and insufficient exercise may appear as excessively fast or slow running speed, over-steep inclination, excessive and insufficient exercise time and improper speed variance, inclination, and exercise time. Appropriate exercise shall be precisely in harmony with the user's individual physical fitness.

Also, the present invention further comprises a synchronous storage step, which continuously monitors the user's physical information such as body weight and blood pressure values during operation of the exercise apparatus and submits such information to said external information unit for establishing said personal information.

Thereby, the next time the user exercises with the apparatus, it is preprogrammed with the exercise mode that best meets the user's demand.

It is to be noted that in addition to the aforesaid treadmill, the present invention can be also applied to a therapeutic exercise machine or other exercise apparatus, such as an elliptical bike.

Although a particular embodiment of the invention has been described in detail for purposes of illustration, various modifications and enhancements may be made without departing from the spirit and scope of the invention.

## Claims

1. A method of programming an electrical human exercise apparatus which comprises the steps of establishing an external information unit, processing said information and executing a corresponding exercise mode, wherein,
establishing an external information unit is to established containing a user's personal information;
processing said information is the user's personal information for the external information unit and simultaneously processing it for outputting at least one exercise mode; and
executing a corresponding exercise mode to drive the apparatus to operate according to a selected exercise mode.

2. The method of programming human electrical exercise apparatus as claimed in claim 1, wherein establishing an external information unit further comprises a direct input step and an indirect input step wherein the direct input step is related to establishing the user's personal information in advance while the indirect input step is for the user to input his personal information item-by-item using either of said input steps to provide personal information to the apparatus for further processing.

3. The method of programming human electrical exercise apparatus as claimed in claim 2, wherein the direct input of the external information unit is performed through a portable storage device which is pre-loaded with the user's personal information and a transmitting interface which is capable of receiving said information in said portable storage device for further processing.

4. The method of programming human electrical exercise apparatus as claimed in claim 3, wherein the portable storage device is a SIM card, a USB flash disk or a memory card.

5. The method of programming human electrical exercise apparatus as claimed in claim 4, wherein the memory card is selected from the group consisting of SD card, MMC card, CF card, MS card, SM card and XD card.

6. The method of programming human electrical exercise apparatus as claimed in claim 1, wherein the apparatus is an exercise treadmill.

7. The method of programming human electrical exercise apparatus as claimed in claim 1, wherein the apparatus is a therapeutic exercise machine.

8. The method of programming human electrical exercise apparatus as claimed in claim 1, which further comprises an adjusting step so that the user can select the exercise mode directly or adjust the exercise mode according to the adjusting step and then select the exercise mode.

9. The method of programming human electrical exercise apparatus as claimed in claim 1 which further comprises a synchronous storage step that continuously monitors the user's physical information whilst operating the apparatus and submits such information to said external information unit for establishing said personal information.
